(19) Europäisches Patentamt
European Patent Office
Office européen des brevets

(11) EP 0 635 472 B1

(12) **EUROPÄISCHE PATENTSCHRIFT**

(45) Veröffentlichungstag und Bekanntmachung des Hinweises auf die Patenterteilung:
**30.07.1997 Patentblatt 1997/31**

(51) Int. Cl.$^6$: **C07C 51/235**, C07C 59/153, B01J 23/652

(21) Anmeldenummer: **94106105.3**

(22) Anmeldetag: **20.04.1994**

(54) **Katalysator für die Herstellung von Glyoxylsäure durch katalytische Oxidation von Glyoxal und Verfahren zu seiner Herstellung**

Catalyst for the production of glyoxylic acid by catalytic oxidation of glyoxal and process for the production of this catalyst

Catalyseur pour la production d'acide glycoxylique par oxydation catalytique du glyoxal et procédé pour la production de ce catalyseur

(84) Benannte Vertragsstaaten:
**CH DE FR GB IT LI NL SE**

(30) Priorität: **21.07.1993 DE 4324442**

(43) Veröffentlichungstag der Anmeldung:
**25.01.1995 Patentblatt 1995/04**

(73) Patentinhaber: **Degussa Aktiengesellschaft 60311 Frankfurt (DE)**

(72) Erfinder:
• **Burmeister, Roland Dr.**
  **D-63826 Geiselbach (DE)**
• **Deller, Klaus Dr.**
  **D-63512 Hainburg (DE)**
• **Despeyroux, Bertrand Dr.**
  **F-78 112 Fourqueux (FR)**
• **Hatté, Christine**
  **F-80450 Camon (FR)**

(56) Entgegenhaltungen:
**EP-A- 0 191 373**       **EP-A- 0 438 948**

**Beschreibung**

Die Erfindung betrifft einen Platin auf Kohlenstoff als Träger enthaltenden Katalysator für die katalytische Oxidation von Glyoxal zu Glyoxylsäure mit guter Aktivität, Selektivität und Ausbeute.

Glyoxylsäure wird großtechnisch durch Oxidation von Glyoxal mit starken Säuren wie Salpetersäure gewonnen. Elektrochemische Verfahren werden auch in der Literatur beschrieben.

Die EP 0 438 948 A1 beschreibt die direkte katalytische Oxidation von Glyoxal mittels heterogenen Trägerkatalysatoren zu Glyoxylsäure. Im dortigen Beispiel 1 wird ein optimierter Katalysator präpariert, indem erstens der Aktivkohleträger nach HCl-Wäsche bei 500° C in einem Luftstrom partiell verbrannt wird, was zu einer Erhöhung seiner spezifischen Trägeroberfläche führt und daß zweitens der so behandelte Träger mit einer wäßrigen Lösung von Hexachloroplatinsäure imprägniert, mit Formaldehyd behandelt, mit Kaliumhydroxid umgesetzt, anschließend gewaschen und getrocknet wird. Der so erhaltene Katalysator weist eine homogene Verteilung an Platin-Teilchen im Bereich von 1,5 bis 2 nm auf.

Der Einsatz des nach EP 0 438 948 hergestellten, 4,2 % Platin enthaltenden, Katalysators bei der katalytischen Oxidation von Glyoxal führt zu einer Ausbeute von 73 % und einer Selektivität von 79,3 % an Glyoxylsäure.

Dabei wird das Verhältnis vom eingesetzten Katalysator zum eingesetzten Glyoxal mit rd. 43 Gew.-% angegeben. Für die im Beispiel 1 angegebene Reaktionszeit von 110 Minuten kann eine Katalysatoraktivität von 1,24 mmol Glyoxylsäure/mmol Platin/Minute berechnet werden.

Die partielle Verbrennung des Aktivkohleträgers und die weiteren Schritte der Katalysatorpräparation sind scheinbar wesentlich für die Einstellung der platinhaltigen, katalytisch aktiven Phasen von EP 0 438 948.

Leider sind entscheidende Schritte der Katalysatorherstellvorschrift nicht nachvollziehbar. Obwohl die partielle Aktivkohle-Verbrennung ein wesentlicher Schritt dieser Katalysatorpräparation ist, fehlt dazu jede Angabe der Einsatz- sowie der resultierenden (nach partieller Verbrennung) Aktivkohlemenge. Darüber hinaus sind viele Schritte dieser Katalysatorpräparation sehr aufwendig. Beispielhaft sei hier die Umsetzung der Aktivkohle während 24 Stunden mit Salzsäure genannt.

Es ist eine Aufgabe der vorliegenden Erfindung, einen Katalysator für die Oxidation von Glyoxal zu Glyoxylsäure anzugeben, der sich gegenüber den Katalysatoren aus dem Stand der Technik durch einen geringeren Edelmetallbedarf und eine daraus resultierende verbesserte Wirtschaftlichkeit auszeichnet. Eine weitere Aufgabe der Erfindung hat ein Herstellverfahren für diesen Katalysator zum Gegenstand.

Die erste Aufgabe der Erfindung wird gelöst durch einen Platin auf Aktivkohle als Träger enthaltenden Katalysator für die katalytische Oxidation von Glyoxal zu Glyoxylsäure.

Der Katalysator ist dadurch gekennzeichnet, daß er noch Molybdän und/oder Cer als Modifikatoren enthält und daß Plattin, bezogen auf den Träger, in einer Menge von 0,1 bis 5,0, vorzugsweise 0,5 bis 3,5 Gew.-% vorliegt und das Gewichtsverhältnis zwischen dem Plattin und dem gleichzeitig anwesenden Molybdän und/oder Cer 1 : 1 bis 1 : 20, vorzugsweise 1 : 1 bis 1 : 5, beträgt und daß Platin und Molybdän und/oder Cer auf dem Träger fein verteilt sind.

Der Katalysator besitzt besonders günstige Eigenschaften, wenn der mittlere Teilchendurchmesser der Aktivkohle 15 bis 30 μm beträgt, ihre spezifische Oberfläche größer als 500 $m^2$/g und ihr Gesamtporenvolumen größer als 0,5 ml/g sind.

Die zweite Aufgabe der Erfindung wird gelöst durch ein Verfahren zur Herstellung eines Katalysators, durch gleichzeitiges Ausfällen einer Plattin- und einer Molybdän- und/oder Cerverbindung auf den Träger aus Aktivkohle, Reduzieren, Filtrieren und Waschen des Produktes.

Das Verfahren ist dadurch gekennzeichnet, daß man eine wäßrige Suspension mit 5 bis 30 Gew.-% Aktivkohle herstellt, daß man Plattin und zusätzlich Molybdän und/oder Cer in Form einer Lösung ihrer wasserlöslichen Verbindungen der Suspension zugibt, wobei der Gehalt dieser Lösung an Platin und Molybdän und/oder Cer entsprechend der gewünschten Beladung der eingesetzten Menge Aktivkohle bemessen ist, daß man diese Mischung aus Suspension und Lösung unter Rühren auf 70 bis 100° C erwärmt, daß man Platin und Molybdän und/oder Cer in Form ihrer schwer löslichen Verbindungen gleichzeitig durch Zugabe einer Base auf der Aktivkohle niederschlägt und daß man anschließend bei unveränderter Temperatur durch Zugabe eines Reduktionsmittels reduziert.

Geeignete wasserlösliche Verbindungen für die Herstellung des Katalysators sind Hexachloroplatinsäure, Molybdatophosphorsäure und Cerchlorid. Als Base zum Ausfällen der Metallverbindungen können Natriumcarbonat, Natronlauge oder Kalilauge eingesetzt werden. Für die Reduktion der ausgefällten Metallverbindungen sind Hydrazin, Natriumformiat, Natriumboranat oder Formaldehyd geeignet, wobei bevorzugt Formaldehyd verwendet wird.

Die Imprägnierung der Aktivkohle mit den Metallverbindungen kann entweder wie oben beschrieben durch gleichzeitige Ausfällung beider Metallkomponenten aus der Lösung erfolgen oder nacheinander. Bei getrennter Ausfällung wird bevorzugt die Aktivkohle zunächst mit Plattin und in einem zweiten Prozeßschritt mit den Molybdän- und/oder Cersalzen imprägniert.

In den folgenden Beispielen 1 bis 6 und Vergleichsbeispielen 1 bis 4 wurden die Leistungsdaten einiger erfindungsgemäßer Katalysatoren und von Vergleichskatalysatoren aus dem Stand der Technik bei der katalytischen Oxidation von Glyoxal zu Glyoxylsäure ermittelt. Bei den gemessenen Leistungsdaten der Katalysatoren handelte es sich um Umsatz, Selektivität, Ausbeute und Aktivität.

Bei der katalytischen Oxidation von Glyoxal entstehen neben dem gewünschten Produkt (Glyoxylsäure) als Überoxidationsprodukte Oxalsäure, Glycolsäure und andere Nebenprodukte. Die Konzentration der Glyoxylsäure in der Reaktionslösung nimmt mit steigender Reaktionszeit zunächst zu und fällt nach Durchschreiten eines Maximums wegen Überoxidation der schon gebildeten Glyoxylsäure zu Oxalsäure wieder ab. Die Konzentration der unerwünschten Nebenprodukte, insbesondere der Oxalsäure, steigt mit zunehmender Reaktionszeit an.

Umsatz, Selektivität, Ausbeute und Aktivität wurden deshalb jeweils für den Zeitpunkt der maximalen Konzentration der Glyoxylsäure gemäß folgender Definitionsgleichungen ermittelt:

$$\text{Umsatz} = \frac{\text{Umgesetze Menge Glyoxal [mmol/l]}}{\text{Eingesetzte Menge Glyoxal [mmol/l]}} \cdot 100\,\%$$

$$\text{Selektivit.} = \frac{\text{Gebildete Menge Glyoxylsäure [mmol/l]}}{\text{Umgesetzte Menge Glyoxal [mmol/l]}} \cdot 100\,\%$$

$$\text{Ausbeute} = \frac{\text{Gebildete Menge Glyoxylsäure [mmol/l]}}{\text{Eingesetzte Menge Glyoxal [mmol/l]}} \cdot 100\,\%$$

$$\text{Aktivität} = \frac{\text{Gebildete Menge Glyoxylsäure [mmol]}}{\text{Eingesetzte Menge Platin [mmol]} \cdot T_{max}\,[\text{min}]}$$

$T_{max}$ bezeichnet dabei die Reaktionszeit bis zum Erreichen der maximalen Glyoxylsäure-Konzentration.

Katalysatorherstellung

Für die Katalysatoren der Beispiele und Vergleichsbeispiele kam dieselbe Aktivkohle als Katalysatorträger zum Einsatz. Sie hatte folgende Eigenschaften:

spezifische Oberfläche: 1500 $m^2$/g nach ASTM-D-3663
Gesamtporenvolumen: 1,5 ml/g nach ASTM-D-4284
mittlere Korngröße: 22 µm
Restaschegehalt: < 2 %

Zur Herstellung der Katalysatoren wurde zunächst eine wäßrige Aktivkohle Suspension hergestellt. Dazu wurden jeweils 100 g Aktivkohle (Trockengewicht), vermindert um das Gewicht der aufzubringenden katalytischen Komponenten, in destilliertes Wasser eingerührt. Für einen Pt/C-Katalysator mit 5 Gew.-% Platin-Beladung kamen also 95 g Aktivkohle zum Einsatz.

Die Imprägnierlösungen mit den katalytisch aktiven Komponenten wurden in ihrer Konzentration und Menge so bemessen, daß ihr Gehalt an den katalytischen Komponenten genau der notwendigen Beladung der eingesetzten Menge Aktivkohle entsprach. Für die Herstellung eines 5 Gew.%igen Pt/C-Katalysators wurde also eine Imprägnierlösung mit einem Gehalt von 5 g Platin angesetzt.

Im Falle der mit Molybdän oder Cer modifizierten Katalysatoren wurden Imprägnierlösungen angesetzt, die sowohl die Platinvorstufen als auch die Vorstufen der Modifikatoren enthielten, um sie gemeinsam auf den Aktivkohleträger abzuscheiden.

Folgende Vorstufen wurden verwendet:

| für Platin: | Hexachloroplatinsäure | $H_2PtCl_6$ |
| für Molybdän: | Molybdatophosphorsäure | $H_3P(Mo_3O_{10})_4$ |
| für Cer: | Cerchlorid | $CeCl_3$ |
| für Blei: | Bleiacetat | $Pb(CH_3CO_2)_2$ |

Blei wurde als Modifikator in Vergleichsbeispiel 4 eingesetzt.

Zur Abscheidung der katalytischen Komponenten auf dem Aktivkohleträger wurden die Imprägnierlösungen in die zuvor hergestellte wäßrige Aktivkohle-Suspension eingerührt.

Dann wurde die Suspension auf 80° C erwärmt und zur Abscheidung der katalytischen Komponenten 10 %ige Natronlauge eingeführt (ca. 75 ml Natronlauge für 100 g eines Katalysators mit 5 Gew.-% Platin). Die katalytischen Komponenten wurden anschließend durch Zugabe einer 37 %igen Formaldehydlösung (2,5 ml für 100 g Katalysator mit 5 Gew.-% Platin) unter Beibehaltung der Temperatur von 80° C reduziert. Nach der Reduktion wurde der fertige Katalysator über eine Nutsche abfiltriert und mit destilliertem Wasser gewaschen.

Zur Bestimmung der Leistungsdaten wurden die Katalysatoren im nassen Zustand eingesetzt.

Katalysator-Prüfung

Die Oxidation von Glyoxal zu Glyoxylsäure wurde in einem 250 ml Rührreaktor mit Begasungsrührer, Thermometer, Alkalidosierung, pH-Meßelektrode und Sauerstoffzufuhr durchgeführt.

Dazu wurden jeweils 90 ml einer wäßrigen Glyoxal-Lösung mit 0,523 g Glyoxal (entsprechend 0,1 mol Glyoxal/1 bzw. 9 mmol Glyoxal) zusammen mit 0,225 g des zu prüfenden Katalysators in den Reaktor eingefüllt. (Die gewählten Einsatzmengen von Katalysator und Glyoxal ergeben die selben Gewichtsverhältnisse wie in Beispiel 2 der EP 0 438 948 A1).

Der Sauerstoff für die Oxidation wurde durch den Begasungsrührer in der Lösung verteilt. Der Gasdruck betrug 10 mbar über Normaldruck, die Reaktionstemperatur 30° C.

Das entstehende Reaktionsprodukt Glyoxylsäure wurde laufend durch Zutropfen von 10 Gew.-%iger Natronlauge neutralisiert. Der pH-Wert der Reaktionslösung konnte dadurch auf einen wert von 7,7 konstant gehalten werden.

Der zeitliche Reaktionsverlauf wurde durch regelmäßige Probenahmen verfolgt. Die Proben wurden mittels Ionenchromatografie und HPLC analysiert.

Die Ergebnisse der Meßreihen sind in der Tabelle 1 aufgelistet. In Spalte 1 sind die Zusammensetzungen der geprüften Katalysatoren angegeben. Dabei bezeichnet zum Beispiel die Schreibweise 5 % Pt - 5 % Mo/C einen mit Molybdän modifizierten Platin-Katalysator auf Aktivkohle mit 5 Gew.-% Platin und 5 Gew.-% Molybdän. Das Kürzel VB kennzeichnet die Vergleichsbeispiele nach dem Stand der Technik, während das Kürzel B die erfindungsgemäßen Beispiele bezeichnet.

Abgesehen von Beispiel B6 kamen stets nur 0,225 g Katalysator zum Einsatz. In Beispiel B6 wurde die Katalysatormenge auf das fünffache gesteigert, d. h. auf 1,125 g.

In Spalte 2 ist die Zeit bis zum Erreichen der maximalen Glyoxylsäure-Konzentration angegeben. Die Angaben zu Umsatz, Selektivität, Ausbeute und Aktivität wurden zu diesem Zeitpunkt ermittelt.

Tabelle 1 zeigt, daß die erfindungsgemäß mit Molybdän modifizierten Katalysatoren der Beispiele B1 und B2 deutlich bessere Selektivitäten, Ausbeuten und Aktivitäten aufweisen als die Vergleichskatalysatoren der Vergleichsbeispiele VB1 und VB2.

Das Vergleichsbeispiel VB4 macht deutlich, daß Blei als Modifikator für die hier betrachtete Reaktion völlig ungeeignet ist, obwohl Blei in anderen Fällen zur Modifizierung von Pt/C-Katalysatoren verwendet wird. Die HPLC-Analyse zeigte in diesem Fall, daß die schlechte Aktivität darauf zurückzuführen ist, daß Glyoxal an diesem Katalysator bevorzugt zu Oxalsäure weiter oxidiert wird.

Beispiele B4, B5 und B6 zeigen, daß mit Cer modifizierte Katalysatoren eine wesentlich verbesserte Selektivität bei der Oxidation von Glyoxalsäure zu Glyoxylsäure aufweisen.

Tabelle 1

| Beispiel | $T_{max}$ [min] | Umsatz [ % ] | Selektivität [ % ] | Ausbeute [ % ] | Aktivität $\dfrac{\text{mmol Glyoxylsäure}}{\text{mmol Platin} \cdot \text{min}}$ |
|---|---|---|---|---|---|
| VB1:  5 % Pt/C | 5 | 89 | 58 | 52 | 16 |
| VB2:  2,5 % Pt/C | 18 | 91 | 62 | 56 | 10 |
| VB3:  1 % Pt/C | 80 | 77 | 69 | 53 | 5 |
| VB4:  5 % Pt-5 % Pb/C | 10 | 70 | 14 | 10 | 1,6 |
| B1:  5 % Pt-5 % Mo/C | 3 | 83 | 70 | 58 | 30 |
| B2:  2,5 % Pt-5 % Mo/C | 4 | 90 | 68 | 61 | 48 |
| B3:  1 % Pt-5 % Mo/C | 75 | 80 | 65 | 52 | 5 |
| B4:  1 % Pt-5 % Ce/C | 65 | 50 | 80 | 40 | 5 |
| B5:  1 % Pt-10 % Ce/C | 120 | 85 | 72 | 61 | 4 |
| B6:  1 % Pt-5 % Ce/C fünffache Kat.-Menge | 3,5 | 90 | 73 | 66 | 29 |

VB: Vergleichsbeispiel        B: Beispiel

Patentansprüche

1. Katalysator, der Platin und Aktivkohle als Träger enthält, für die katalytische Oxidation von Glyoxal zu Glyoxylsäure,

**dadurch gekennzeichnet,**
daß der Katalysator noch Molybdän und/oder Cer als Modifikatoren enthält und daß Platin, bezogen auf den Träger, in einer Menge von 0,1 bis 5,0, vorzugsweise 0,5 bis 3,5 Gew.-% vorliegt und das Gewichtsverhältnis zwischen dem Platin und dem gleichzeitig anwesenden Molybdän und/oder Cer 1 : 1 bis 1 : 20, vorzugsweise 1 : 1 bis 1 : 5, beträgt und daß Platin und Molyodän und/oder Cer auf dem Träger fein verteilt sind.

2. Katalysator nach Anspruch 1,
**dadurch gekennzeichnet,**
daß der mittlere Teilchendurchmesser der Aktivkohle 15 - 30 μm beträgt, ihre spezifische Oberfläche größer als 500 $m^2$/g und ihr Gesamtporenvolumen größer als 0,5 ml/g sind.

3. Verfahren zur Herstellung des Katalysators nach Anspruch 2 durch Ausfällen einer Platinverbindung auf den Träger aus Aktivkohle, Reduzieren, Filtrieren und Waschen des Produktes,
**dadurch gekennzeichnet,**
daß man eine wäßrige Suspension mit 5 bis 30 Gew.-% Aktivkohle herstellt, daß man Platin und zusätzlich Molybdän und/oder Cer in Form einer Lösung ihrer wasserlöslichen Verbindungen der Suspension zugibt, wobei der Gehalt dieser Lösung an Platin und Molybdän und/oder Cer entsprechend der gewünschten Beladung der eingesetzten Menge Aktivkohle bemessen ist, daß man diese Mischung aus Suspension und Lösung unter Rühren auf 70 bis 100°C erwärmt, daß man Platin und Molybdän und/oder Cer in Form ihrer schwer löslichen Verbindungen gleichzeitig durch Zugabe einer Base auf der Aktivkohle niederschlägt und daß man anschließend bei unveränderter Temperatur durch Zugabe eines Reduktionsmittels reduziert.

4. Verfahren zur Herstellung von Glyoxylsäure durch Oxidation von Glyoxalsäure in einem wäßrigen Medium unter Zufuhr von Sauerstoff unter Verwendung eines Katalysators nach Anspruch 1.

**Claims**

1. Catalyst which contains platinum and, as a support, carbon, for the catalytic oxidation of glyoxal to glyoxylic acid, characterised in that the catalyst contains in addition molybdenum and/or cerium as modifiers, and that platinum is present in a quantity of from 0.1 to 5.0 wt.%, preferably 0.5 to 3.5 wt.%, referred to the support, and the weight ratio of the platinum to the molybdenum and/or cerium simultaneously present is from 1 : 1 to 1 : 20, preferably 1 : 1 to 1 : 5 and that platinum and molybdenum and/or cerium are finely divided on the support.

2. Catalyst according to claim 1,
characterised in that the average particle diameter of the activated carbon is from 15 to 30 μm, the specific surface thereof is greater than 500 $m^2$/g and the total pore volume thereof is greater than 0.5 ml/g.

3. Process for the preparation of the catalyst according to claim 2 by precipitation of a platinum compound onto the support consisting of activated carbon, reduction, filtration and washing of the product,
characterised in that an aqueous suspension containing 5 to 30 wt.% of activated carbon is prepared, that platinum and in addition molybdenum and/or cerium in the form of a solution of their water-soluble compounds are added to the suspension, the content of platinum and of molybdenum and/or cerium in this solution being calculated according to the required loading of the quantity of activated carbon used, that this mixture of suspension and solution is heated, with stirring, to 70 to 100°C, that platinum and molybdenum and/or cerium in the form of their sparingly soluble compounds are simultaneously deposited onto the activated carbon by addition of a base and that reduction is then carried out at unchanged temperature by addition of a reducing agent.

4. Process for the preparation of glyoxylic acid by oxidation of glyoxalic acid in an aqueous medium with the supply of oxygen and with the use of a catalyst according to claim 1.

**Revendications**

1. Catalyseur, qui contient du platine et du charbon comme support, pour l'oxydation catalytique de glyoxal en acide glyoxylique,
caractérisé en ce que
le catalyseur contient en plus du molybdène et/ou du cérium comme modifiants et que le platine, par rapport au support, est présent en une quantité de 0,1 à 5,0, de préférence 0,5 à 3,5 % en poids et que le rapport pondéral, entre le platine et le molybdène et/ou le cérium simultanément présent, représente 1:1 à 1:20, de préférence 1:1 à 1:5 et que le platine et le molybdène et/ou le cérium sont finement dispersés sur le support.

2. Catalyseur selon la revendication 1,
   caractérisé en ce que
   le diamètre moyen de particule du charbon actif représente 15-30 µm, sa surface spécifique est supérieure à 500 m$^2$/g et son volume total de pores est supérieur à 0,5 ml/g.

3. Procédé de préparation du catalyseur selon la revendication 2, par précipitation d'un composé du platine sur le support de charbon actif, réduction, filtration et lavage du produit,
   caractérisé en ce qu'
   on prépare une suspension aqueuse de 5 à 30 % en poids de charbon actif, en ce qu'on ajoute à la suspension du platine et en plus du molybdène et/ou du cérium sous forme d'une solution de leurs composés hydrosolubles, où la teneur de cette solution en platine et molybdène et/ou cérium est déterminée en fonction de la charge souhaitée pour la quantité mise en oeuvre de charbon actif, qu'on chauffe ce mélange de suspension et de solution en agitant entre 70 et 100°C, qu'on précipite le platine et le molybdène et/ou le cérium simultanément sous forme de leurs composés peu solubles par addition d'une base sur le charbon actif et qu'ensuite, on réduit à la même température par addition d'un réducteur.

4. Procédé de préparation d'acide glyoxylique par oxydation d'acide glyoxalique dans un milieu aqueux, en ajoutant de l'oxygène, en utilisant un catalyseur selon la revendication 1.